# EUROPEAN PATENT SPECIFICATION

(11) **EP 0 149 175 B1**
(45) Date of publication and mention of the grant of the patent: **27.01.1993**
(21) Application number: 84115690.4
(22) Date of filing: 18.12.1984
(51) Int. Cl.: A01N 25/04, A01N 43/40, A61K 7/06

(54) **Dispersions of antifungus agents and antifungal hair treatment compositions**
Dispersionen pilzhemmender Mittel und pilzhemmende Mittel zur Behandlung der Haare
Dispersion d'agents antifongiques et compositions antifongiques pour le traitement des cheveux

(30) Priority: 27.12.1983 JP 248353/83
(43) Date of publication of application: 24.07.1985
(73) Proprietor: Kao Corporation, Chuo-Ku Tokyo 103 (JP)
(72) Inventor: Takaya, Susumu, Funabashi-shi, Chiba-ken (JP); Hirota, Hajime, Meguro-ku, Tokyo (JP)
(74) Representative: Wächtershäuser, Günter, Prof. Dr.

(56) References cited:
- BE-A- 702 495
- US-A- 3 723 325
- US-A- 3 761 418

## Description

### i) Field of The Invention:

This invention relates to aqueous dispersions of antifungus agents and antifungal hair treatment compositions in which zinc salts of 2-mercaptopyridine-N-oxide are stably dispersed.

### ii) Description of The Prior Art:

Metal salts of 2-mercaptopyridine-N-oxide (hereinafter referred to simply as "Mept" compounds) are known to be effective as germicides and are widely utilized not only as ordinary germicides, but also as dandruff removers being added to shampoos. The metals of the Mept compounds are calcium, magnesium, barium, strontium, zinc, cadmium, tin, zirconium and the like. Of these, zinc salts are widely used.

However, these Mept compounds are sparingly soluble in water and high in specific gravity, e.g. the zinc salt (hereinafter referred to simply as "Zpt compound") has a solubility in water of 15 ppm at 25°C and specific gravity of 1.8. Accordingly, they have been commercially sold in the form of powder or aqueous dispersions. Because of the high specific gravity, the Mept compounds are apt to coagulate, settle and separate even when suspended or dispersed in solvents.

Commercially sold aqueous dispersions of Mept compounds have been heretofore prepared by shearing the Mept compound in the presence of dispersants. However, some dispersions may settle and solidify at the bottom of a container during storage. Even with relatively stable dispersions, they may disadvantageously coagulate and separate by incorporation of salts or freezing.

US-A-3 723 325 and US-A-3 761 418 as well as BE-A-702 495 describe detergent compositions, including shampoos, containing a water-insoluble particulate substance and at least one water-soluble cationic nitrogen-containing polymer which serves to enhance the deposition and retention of said particulate substance on surfaces washed with the detergent composition.

At present, shampoos and rinses comprising Mept compounds are put on the market. Several methods of stably adding Mept compounds to shampoo or rinse compositions are known including, for example, a method of adding highly viscous polymers such as cross-linked polyacrylates (Japanese Patent Publication No. 49-49117) and a method of adding acrylic acid/acrylate copolymers (Japanese Patent Publication No. 54-16951). However, these methods have the drawback that limitation is placed on the type of surface active agent and the pH range used to stably disperse Mept compounds.

### SUMMARY OF THE INVENTION

In view of the above, the present inventors have made intensive studies in order to overcome the drawback and, as a result, found that when specific types of polymer compounds are used to disperse Mept compounds in water, the resulting dispersion is very stable under ordinary preservation conditions and resistant to salts and, when once frozen and then melted, remains unchanged. To a surprise, it was also found that when the thus prepared aqueous dispersion of the Mept compounds was added to bases of hair treatments such as shampoos, hair rinses and hair lotions, the dispersed state of the Mept compound is stably maintained without involving any limitation as mentioned above.

The present invention is accomplished based on the above findings. According to one aspect of the present invention, there is provided a dispersion of an antifungus agent comprising (A) a zinc salt of 2-mercaptopyridine-N-oxide and (B) a condensation product of dipropylenetriamine and polyethyleneglycol/epichlorohydrin/longchain alkylamine of the type commercially sold under the name of polyquart H®.

According to another aspect to the invention, there is also provided an antifungal hair treatment composition which comprises a base for hair treatment and a dispersion of an antifungus agent as defined above.

### DETAILED DESCRIPTION OF THE INVENTION AND PREFERRED EMBODIMENTS

The zinc salt of 2-mercaptopyridine-N-oxide which is (A) ingredient of the present invention is represented by the following general formula 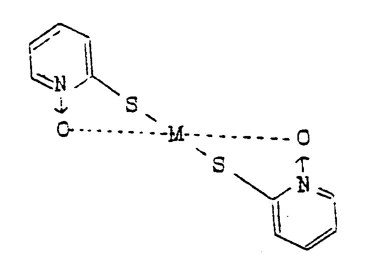
in which M represents a zinc atom.

Zinc salt of 2-mercaptopyridine-N-oxide is hereinafter referred to simply as zinc pyrithione. The salt is commercially solo in the form of a powder having an average size of about 0.5 to 1.5 microns or as an aqueous dispersion.

The condensation resins which are (B) ingredient of the invention are curable condensation products as defined in Claim 1.

Preferable condensation resins are mentioned below, said resins are mentioned only for illustrative purposes and are not ingredient (B) of the present invention as defined in claim 1.
(i) Reaction product of dipropylenetriamine, bis-chlorohydrin ether of polyoxyethylene glycol having an average molecular weight of about 1000 and epichlorohydrin.
(ii) Reaction product of dipropylenetriamine, bis-chlorohydrin ether of polyoxyethylene glycol having an average molecular weight of about 1000 and bis-chlorohydrin ether of polyoxyethylene glycol having an average molecular weight of 600.
(iii) Reaction product of diethylenetriamine, ethoxylated ethylene chlorohydrin and epichlorohydrin.
(iv) Reaction product of dipropylenetriamine ethoxylated glycerine chlorohydrin ether and epichlorohydrin.
(v) Reaction product of triethylenetetramine, bis-chlorohydrin ether of polyoxyethylene glycol having an average molecular weight of about 1000 and bis-chlorohydrin ether of polyoxyethylene glycol having an average molecular weight of about 600.
(vi) Reaction product of dipropylenetriamine and bis-chlorohydrin ether of polyoxyethylene glycol having an average molecular weight of about 600.
(vii) Reaction product of dipropylenetriamine and bis-chlorohydrin ether of polyoxyethylene glycol having an average molecular weight of about 200.
(viii) Reaction products of the ingredients, respectively, used in (i) to (vii) and alkyl or alkyleneamines having 10 to 24 carbon atoms.

The ratio of the chlorine atoms to the hydrogen atoms of amino group in the reaction products of (i) to (viii) is preferred to be in the range of 4:5 to 7:5.

The condensation resin ingredient (B) of the present invention is commercially sold under the name of Polyquart H from Henkel Co., Ltd., as a 50% aqueous solution.

The dispersion of antifungus agent generally comprises 0.0015 to 60 wt% (hereinafter referred simply as %), preferably 0.1 to 50%, of (A) ingredient and (B) ingredient at a ratio by weight of 1/0.01 to 1/10, preferably 1/0.01 to 1/5. The balance should preferably be an aqueous medium such as water or a lower alcohol/water.

The dispersion is prepared by a method in which a powder of (A) ingredient is added to a 50% solution of (B) ingredient or a diluted solution thereof and agitated to give a uniform mixture, or by a method in which a dispersion of (A) ingredient is added to a 50% solution of (B) ingredient or a diluted solution thereof under agitation and agitated to obtain a uniform mixture. The mixing is carried out using ordinary agitated mixers such as propeller agitator apparatus, homogenizer mixers, sand mills and the like.

The hair treatments according to another embodiment of the invention include, for example, shampoos, rinses, hair lotions and the like. These treatments are obtained by adding the dispersion of antifungus agent to hair treatment bases under agitation and uniformly mixing them. The amount of the dispersion is determined such that (A) ingredient in the final composition is in the range of 0.01% to 10%, preferably 0.05 to 5%.

The (A) and (B) ingredients should be added to bases for the hair treatment in the form of a dispersion. Even though the (A) and (B) ingredients and the bases are mixed in ordinary techniques, stable antifungal hair treatment compositions cannot be obtained.

The bases for hair treatments may be ones ordinarily used for these purposes. Shampoo compositions are preferable in the practice of the invention. The bases for shampoos include anionic surface active agents, amphoteric surface active agents, nonionic surface active agents, cationic surface active agents and mixtures thereof. Specific examples of these surface active agents are mentioned below.

Anionic surface active agents:
(1) Linear or branched alkylbenzenesulfonates having an alkyl group containing 10 to 16 carbon atoms on the average.
(2) Polyoxyalkylene alkyl ether sulfates having a linear or branched alkyl group having 8 to 20 carbon atoms on the average and added with 0.5 to 8 moles of ethylene oxide and/or propylene oxide in one molecule thereof on the average.
(3) Alkylsulfates having a linear or branched alkyl group having 10 to 20 carbon atoms on the average.
(4) Olefinsulfonates having 10 to 20 carbon atoms in one molecule thereof on the average.
(5) Alkanesulfonates having 10 to 20 carbon atoms in one molecule thereof on the average.
(6) Fatty acid salts having a linear or branched, saturated or unsaturated hydrocarbon chain having 10 to 20 carbon atoms on the average.
(7) Alkylethoxycarboxylates having a linear or branched alkyl group having 10 to 20 carbon atoms and added with 0.5 to 8 moles of ethylene oxide in one molecule thereof on the average.
(8) Alkyl or alkenylsuccinates having an alkyl or alkenyl group having 6 to 20 carbon atoms on the average and partially neutralized salts thereof.
(9) Phosphate active agents of the formula 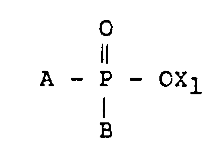 in which A represents 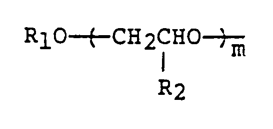 or 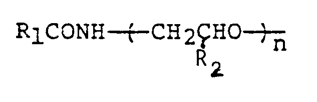 (in which R₁ represents a linear or branched, saturated or unsaturated hydrocarbon group, R₂ represents a hydrogen atom or a methyl group, m is a value of from 0 to 6, and n is a value of 1 to 6), B represents -OX₂ or A, and X₁ and X₂ independently represent a hydrogen or counter ion.
(10) Amino acid surface active agent of the formula 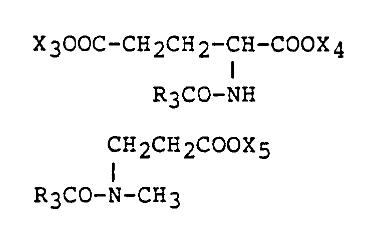 in which R₃ represents an alkyl or alkenyl group having 7 to 21 carbon atoms, X₃, X₄ and X₅ independently represent a hydrogen atom or counter ion.
(11) Acylated polypeptide surface active agents of the formula 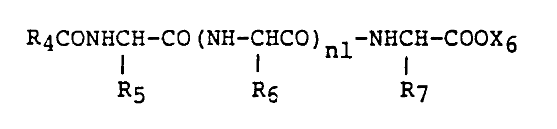 in which R₄ represents an alkyl or alkenyl group having 7 to 21 carbon atoms, R₅, R₆ and R₇ independently represent a side chain of an amino acid, n₁ is a value of 1 to 6, and X₆ represents a hydrogen atom or counter ion.
   The counter ions represented by X₁ to X₆ of these anionic surface active agents generally include ions of alkali metals such as sodium, potassium and the like; alkaline earth metals such as magnesium; ammonium ion and alkanolamine bases having 1 to 3 alkanol groups having 2 or 3 carbon atoms such as, for example, monoethanolamine, diethanolamine, triethanolamine, triisopropylamine and the like.
   Amphoteric surface active agents:
(12) Alkylamine oxides (i) and amidoamine oxides (ii) of the following formulae 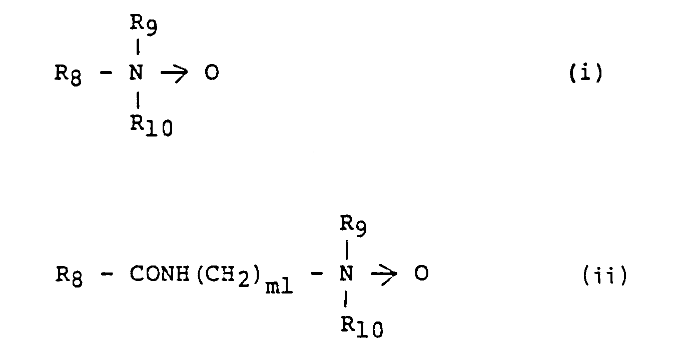 in which R₈ represents an alkyl or alkenyl group having 10 to 20 carbon atoms, R₉ and R₁₀ independently represent an alkyl group having 1 to 3 carbon atoms, and m₁ is an integer of 1 to 4.
(13) Alkyl or sulfobetaines (i) and amido or amidosulfobetaines (ii) of the following formulae 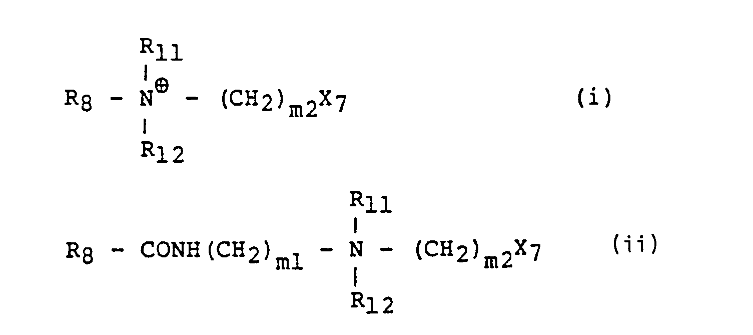 in which R₁₁ and R₁₂ independently represent an alkyl group having 1 to 4 carbon atoms, m₂ is an integer of 1 to 3, X₇ represents a -COO^{⊖} or -SO₃^{⊖} group, m₁ and R₈ have the same meanings as defined before, respectively.
(14) Imidazoline amphoteric surface active agents of the following formula 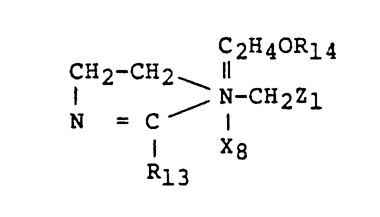 in which R₁₃ is an aliphatic acid residue having 10 to 20 carbon atoms on the average, R₁₄ represents sodium, hydrogen or -CH₂COOM₂, Z₁ represents -COOM₂, -CH₂COOM₂ or 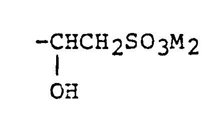 in which M₂ represents sodium, hydrogen or an organic base, X₈ represents a hydroxyl group, an acidic salt, an anionic surface active sulfate or a sulfated product.
(15) Amidoamine amphoteric surface active agents of the formula 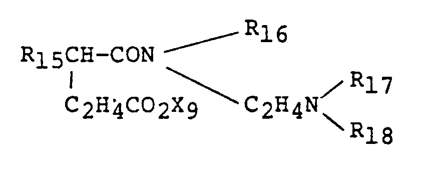 in which R₁₅ represents an alkyl or alkenyl group having 6 to 20 carbon atoms, R₁₆ represents hydrogen, -C₂H₄OH or -C₂H₄OC₂H₄COOX₉, R₁₇ represents -C₂H₂OH, -C₂H₄OC₂H₄COOX₉ or -C₂H₄COOX₉, and R₁₈ represents hydrogen or -C₂H₄COOX₉, X₉ represents hydrogen, alkali metal, ammonium or organic ammonium.
(16) Polyoxyethylene alkylor alkenyl ethers having an alkyl or alkenyl group having 10 to 20 carbon atoms on the average and added with 1 to 20 moles of ethylene oxide.
(17) Polyoxyethylene alkylphenyl ethers having an alkyl group having 6 to 12 carbon atoms on the average and added with 1 to 20 moles of ethylene oxide.
(18) Polyoxypropylene alkyl or alkenyl ethers having an alkyl or alkenyl group ahving 10 to 20 carbon atoms on the average and added with 1 to 20 moles of propylene oxide.
(19) Polyoxybutylene alkyl or alkenyl ethers having an alkyl or alkenyl group having 10 to 20 carbon atoms on the average and added with 1 to 20 moles of butylene oxide.
(20) Nonionic surface active agents having an alkyl or alkenyl group having 10 to 20 carbon atoms on the average and added with 1 to 30 moles, in total, of ethylene oxide and propylene oxide or ethylene oxide and butylene oxide (a ratio of ethylene oxide and propylene oxide or butylene oxide is in the range of 0.1/9.9 to 9.9/0.1).
(21) Higher fatty acid alkanolamides of the following formula and alkylene oxide adducts thereof 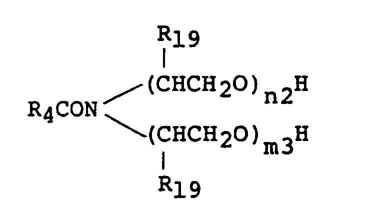 in which R₄ has the same meaning as defined before, R₁₉ is a hydrogen atom or a methyl group, n₂ is an integer of 1 to 3, and m₃ is an integer of 0 to 3.
(22) Fatty acid sucrose esters consisting of fatty acid having 10 to 20 carbon atoms on the average and sucrose.
(23) Fatty acid glycerine monoesters consisting of a fatty acid having 10 to 20 carbon atoms on the average and glycerine.
   Cationic surface active agents:
(24) 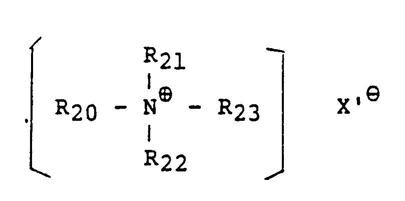 in which at least one of R₂₀, R₂₁, R₂₂ and R₂₃ represents an alkyl or alkenyl group having 8 to 24 carbon atoms and the other represent an alkyl group having 1 to 5 carbon atoms, and X' represents a halogen atom.
(25) 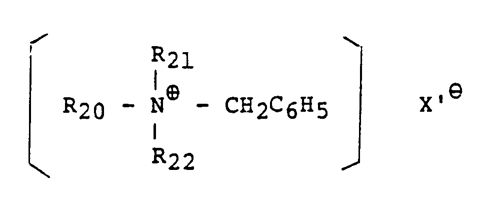 in which R₂₀, R₂₁, R₂₂ and X' have the same meanings as defined before, respectively.
(26) 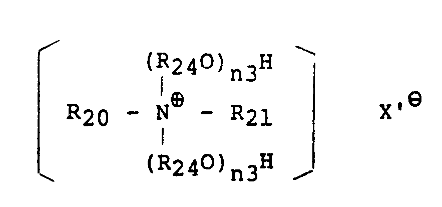 in which R₂₀, R₂₁ and X' have the same meanings as defined before, respectively, n₃ is an integer of 1 to 20, and R₂₄ represents an alkylene group having 2 to 3 carbon atoms.

Of these surface active agents, the agents of (1), (2), (3), (4), (5), (6), (11), (i) and (ii) of (12), (13), (14), (15), (16) and (21) are preferred. These surface active agents may be used singly or in combination. The amount of the surface active agents is generally in the range not less than 5%, preferably from 10 to 40%, of the composition.

The antifungal hair treatment composition may further comprise, aside from the above-described essential ingredients, any arbitrary ingredients ordinarily used for these purposes. Examples of such arbitrary ingredients include: solubilizers such as propylene glycol, glycerine, urea and the like; viscosity modifiers such as ethyl alcohol, isopropyl alcohol, hydroxyethyl cellulose, methyl cellulose, higher alcohols and the like; antimicrobial agents; antiseborrheic agents; keratin-soluble or swelling substances such as sulfur, salicylic acid and enzymes, deodorants, pearling agents, lotionizing agents, perfumes, colorants, UV absorbers, antioxidants, preservatives and the like.

The present invention is described by way of examples and comparative examples.

### Example 1

| | |
|---|---|
| Zinc pyrithione (powder) | 15(%) |
| Polyquart H® (condensation resin of polyglycolpolyamine and long chain alkylamine) | 60 |
| Water | balance |

The zinc pyrithione was added to an aqueous diluted solution of Polyquart H® (60:25) and mixed under agitation to obtain a dispersion of the antifungus agent.

### Example 2

| | |
|---|---|
| Zinc pyrithione (commercially available dispersion A, concentration 50%) | 30(%) |
| Polyquart H® | 30 |
| Water | balance |

A dispersion of the antifungus agent was obtained in the same manner as in Example 1.

The dispersions obtained in Examples 1 and 2 and a commercially sold zinc pyrithione dispersions were tested to determine stability when preserved at room temperature and at 40°C for 1 month and to check a state of each dispersion when once frozen and melted. The results are shown in Table 1 below, from which it will be seen that the dispersions of the present invention are stable when preserved over a long term and give no flocculations when re-melted.

**Table 1**

| | Example 1 | Example 2 | Commercial Product A | Commercial Product B | Commercial Product C |
|---|---|---|---|---|---|
| 40°C | o | o | S | S | S |
| Room Temp. | o | o | o | o | S |
| Frozen and re-melted | o | o | F | F | F |
| Note: o: stable S: separation F: flocculation | | | | | |

### Example 3

| | |
|---|---|
| (1) Sodim Polyoxyethylene(2.5)laurylethersulfate | 15(%) |
| (2) Coconut oil fatty acid diethanolamide | 3 |
| (3) Dispersion of antifungus agent (Example 1) | 7 |
| (4) Perfume | 0.5 |
| (5) Colorant | small amount |
| (6) Citric acid | small amount |
| (7) Water | balance |

In a uniform solution of (1), (2) and (7) was dispersed (3) at room temperature under agitation, followed by adding (4), (5) and (6) to obtain a shampoo composition.

### Example 4

| | |
|---|---|
| Lauryl sulfate triethanolamine salt | 15(%) |
| Dispersion of antifungus agent (Example 2) | 7 |
| Perfume | 0.5 |
| Colorant | small amount |
| Citric acid | small amount |
| Water | balance |

In the same manner as in Example 3, there was obtained a shampoo composition.

### Comparative Example 1

| | |
|---|---|
| (1) Sodium Polyoxyethylene(2.5)laurylethersurfate | 15(%) |
| (2) Coconut oil fatty acid diethanolamide | 3 |
| (3) Zinc pyrithione (powder) | 1 |
| (4) Perfume | 0.5 |
| (5) Colorant | small amount |
| (6) Citric acid | small amount |
| (7) Water | balance |

In a uniform solution of (1), (2) and (7) was uniformly dispersed (3) at room temperature under agitation, followed by adding (4), (5) and (6).

### Comparative Example 2

| | |
|---|---|
| (1) Sodium Polyoxyethylene(2.5)laurylethersulfate | 15(%) |
| (2) Coconut oil fatty acid diethanolamide | 3 |
| (3) Polyquart H | 2 |
| (4) Zinc pyrithione (powder) | 1 |
| (5) Perfume | 0.5 |
| (6) Colorant | small amount |
| (7) Citric acid | small amount |
| (8) Water | balance |

To a uniform solution of (1), (2) and (8) was added (3) under agitation, followed by uniformly dispersing (4) and then adding (5), (6) and (7).

### Comparative Example 3

| | |
|---|---|
| (1) Sodium Polyoxyethylene(2.5)laurylethersulfate | 15(%) |
| (2) Coconut oil fatty acid diethanolamide | 3 |
| (3) Carboxyvinyl polymer | 0.5 |
| (4) Zinc pyrithione (powder) (Commercially sold dispersion A with a concentration of 50%) | 2 |
| (5) Caustic soda | small amount |
| (6) Perfume | 0.5 |
| (7) Colorant | small amount |
| (8) Water | balance |

To a uniform solution of (1) and (8) were added (3) and then (2) at room temperature under agitation. The pH was adjusted to 7 by the use of (5), followed by uniformly dispersing (4) and subsequently adding (6) and (7).

Alternatively, a dispersion of (3) and (4) was first prepared and added to a uniform solution of (1), (2) and (8), followed by adjusting the pH to 7 by the use of (5) and adding (6) and (7).

The thus prepared antifungal shampoo composition was tested to check a stability with time by day and a stability at the time when the composition was frozen and re-melted. The results are shown in Table 2.

The antifungal shampoo compositions of the invention showed good stability without causing flocculation an separation of the zinc pyrithione.

**Table 2**

| | Example 3 | Example 4 | Comparative Example 1 | Comparative Example 2 | Comparative Example 3 |
|---|---|---|---|---|---|
| 50°C | o | o | S | S | S |
| 40°C | o | o | S | S | S |
| Room Temp. | o | o | S | S | S |
| Frozen and re-melted dispersion | o | o | S | S | S |
| Note: o and S have the same meanings as defined with reference to Table 1, respectively. | | | | | |

### Example 5

| Shampoo Composition: | |
|---|---|
| (1) Sodium Polyoxyethylene(2.5)laurylethersulfate | 10(%) |
| (2) Imidazoline amphoteric surface active agent* | 15 |
| (3) Antifungal dispersion (Example 1) | 7 |
| (4) Perfume | 0.5 |
| (5) Colorant | small amount |
| (6) Citric acid | small amount |
| (7) Water | balance |

| | |
|---|---|
| * Miranol C2M® (Miranol Co., Ltd.) | |

In a uniform solution of (1), (2) and (7) was uniformly dispersed (3) at room temperature under agitation, followed by adding (4), (5) and (6) to obtain a shampoo composition.

### Example 6

| Shampoo Composition: | |
|---|---|
| (1) Sodium Polyoxyethylene(2.5)laurylethersulfate | 10(%) |
| (2) Coconut oil fatty acid amidobetaine | 6 |
| (3) Antifungal dispersion (Example 1) | 7 |
| (4) Perfume | 0.5 |
| (5) Colorant | small amount |
| (6) Citric acid | small amount |
| (7) Water | balance |

In a uniform solution of (1), (2) and (7) was uniformly dispersed (3) at room temperature under agitation, followed by adding (4), (5) and (6) to obtain a shampoo composition.

### Example 7

| Shampoo composition: | |
|---|---|
| (1) Triethanolamine salt of lauryl sulfate | 12(%) |
| (2) Monotriethanolamine coconut oil fatty acid acyl-L-glutaminate | 4 |
| (3) Antifungal dispersion (Example 1) | 7 |
| (4) Perfume | 0.5 |
| (5) Colorant | small amount |
| (6) Citric acid | small amount |
| (7) Water | balance |

In a uniform solution of (1), (2) and (7) was uniformly dispersed (3) at room temperature under agitation, followed by adding (4), (5) and (6) to obtain a shampoo composition.

### Example 8

| Shampoo composition: | |
|---|---|
| (1) Sodium Polyoxyethylene(2.5)-laurylethersulfate | 10(%) |
| (2) Sodium salt of lauroylsarcosine | 6 |
| (3) Antifungal dispersion (Example 1) | 7 |
| (4) Perfume | 0.5 |
| (5) Colorant | small amount |
| (6) Citric acid | small amount |
| (7) Water | balance |

In a uniform solution of (1), (2) and (7) was uniformly dispersed (3) at room temperature under agitation, followed by adding (4), (5) and (6) to obtain a shampoo composition.

### Example 9

| Shampoo composition: | |
|---|---|
| (1) Sodium C₁₄₋₁₆ alpha-olefinsulfonate | 14(%) |
| (2) coconut oil fatty acid diethanolamide | 3 |
| (3) Antifungal dispersion (Example 1) | 7 |
| (4) Perfume | 0.5 |
| (5) Colorant | small amount |
| (6) Citric acid | small amount |
| (7) Water | balance |

In a uniform solution of (1), (2) and (7) was uniformly dispersed (3) at room temperature under agitation, followed by adding (4), (5) and (6) to obtain a shampoo composition.

### Example 10

| Hair rinse composition: | |
|---|---|
| (1) Distearyldimethylammonium chloride | 2(%) |
| (2) Cetyl alcohol | 2 |
| (3) Propylene glycol | 3 |
| (4) Antifungal dispersion (Example 1) | 3 |
| (5) Perfume | 0.5 |
| (6) Colorant | small amount |
| (7) Citric acid | small amount |
| (8) Water | balance |

In a uniform solution of (1) and (8) was uniformly dispersed (4) and heated, followed by adding a hot uniform solution of (2) and (3) under agitation, cooling and adding (5), (6) and (7) to obtain a hair rinse composition.

### Example 11

| Dandruff remover: | |
|---|---|
| Zinc pyrithione (commercially sold dispersion A with a concentration of 50%) | 4(%) |
| Polyquart H® | 2.0 |
| Propylene glycol | 5 |
| Ethanol | 10 |
| Perfume | small amount |
| Water | balance |

A dandruff remover was obtained in the same manner as in Example 2. The remover was found to be stable over 1 month at room temperature.

On the contrary, when no Polyquart H® was used, the Zinc pyrithione was recognized to settle in 3 days.

## Claims

1. A dispersion of an antifungus agent characterized by comprising (A) a zinc salt of 2-mercaptopyridine-N-oxide and (B) a condensation product of dipropylenetriamine and polyethyleneglycol/epichlorohydrin/longchain alkylamine of the type commercially sold under the name of polyquart H®.

2. An antifungus hair treatment composition characterized by adding to a hair treatment basis a dispersion of an antifungus agent as defined in Claim 1.

3. The antifungal hair treatment composition according to Claim 2, wherein the hair treatment is a shampoo.

## Patentansprüche

1. Dispersion eines Antifungusmittels, dadurch gekennzeichnet, daß sie (A) ein Zinksalz von 2-Mercaptopyridin-N-oxid und (B) ein Kondensationsprodukt von Dipropylentriamin und Polyethylenglycol/Epichlorhydrin/langkettiges Alkylamin des Typs, der im Handel unter der Bezeichnung Polyquart H^{(R)} erhältlich ist, umfaßt.

2. Antifungus-Haarbehandlungszusammensetzung, dadurch gekennzeichnet, daß einer Haarbehandlungsbasis eine Dispersion eines Antifungusmittels, wie es in Anspruch 1 definiert ist, zugesetzt ist.

3. Antifungus-Haarbehandlungszusammensetzung gemäß Anspruch 2, wobei die Haarbehandlung ein Schampoo ist.

## Revendications

1. Dispersion d'un agent antifongique, caractérisée par le fait qu'elle comprend :
(A) un sel de zinc du mercapto-2 pyridine-N-oxyde ; et
(B) un produit de condensation de dipropylène triamine et de polyéthylène glycol/épichlorhydrine/alkyl amine à longue chaîne du type commercialisé sous la dénomination de Polyquart H®.

2. Composition de traitement capillaire antifongique, caractérisée par l'addition à une base de traitement capillaire, d'une dispersion d'un agent antifongique tel que défini à la revendication 1.

3. Composition de traitement capillaire antifongique selon la revendication 2, dans laquelle le traitement capillaire est un shampooing.
